# EUROPEAN PATENT SPECIFICATION

(11) **EP 3 482 706 B1**
(45) Date of publication and mention of the grant of the patent: **05.10.2022**
(21) Application number: 16908148.6
(22) Date of filing: 06.07.2016
(51) Int. Cl.: A61M 25/10, A61M 25/01, A61B 1/00, A61B 1/018, A61B 17/00, A61B 17/12

(54) **TREATMENT TOOL FOR ENDOSCOPE**
BEHANDLUNGSWERKZEUG FÜR ENDOSKOP
INSTRUMENT DE TRAITEMENT DESTINÉ À UN ENDOSCOPE

(43) Date of publication of application: 15.05.2019
(73) Proprietor: Olympus Corporation, Hachioji-shi, Tokyo 192-8507 (JP)
(72) Inventor: FUKUSHIMA Norichika, Hachioji-shi Tokyo 192-8507 (JP); ONISHI Koji, Hachioji-shi Tokyo 192-8507 (JP); MIYAUCHI Shunsuke, Hachioji-shi Tokyo 192-8507 (JP); YUASA Masaru, Hachioji-shi Tokyo 192-8507 (JP); NITTA Satoshi, Hachioji-shi Tokyo 192-8507 (JP)
(74) Representative: Gunzelmann, Rainer
(86) International application number: PCT/JP2016/069988
(87) International publication number: WO 2018/008104

(56) References cited:
- WO-A1-2015/146259
- WO-A1-2015/146259
- JP-A- 2000 051 361
- JP-A- 2003 299 663
- JP-A- 2006 239 156
- JP-A- 2013 141 588
- JP-A- 2014 124 265
- US-A1- 2009 312 745
- US-A1- 2015 238 736
- US-B1- 6 488 653

## Description

### [Technical Field]

The present invention relates to a treatment tool for an endoscope used in expansion treatment of a stenosis site or an obstruction site in a lumen of a living body.

### [Background Art]

Conventionally, in combination with an endoscope, a procedure for performing dilation treatment or the like of a stenosis site or an obstruction site (hereinafter referred to as "stenosis site or the like") of a digestive tract is performed. In such a procedure, for example, a treatment tool for an endoscope equipped with a balloon is used. Specifically, the treatment tool for an endoscope is inserted into a lumen of a living body along with the endoscope, and the balloon inflated while the balloon is inserted into the stenosis site or the like to dilate the stenosis site or the like. When the treatment tool for an endoscope is disposed to dilate the stenosis site or the like in the lumen of the living body, the balloon may slip against the stenosis site or the like while the balloon is inflated, and the balloon may be removed from a site to be dilated. In this case, since an operator needs to temporarily deflect the balloon first, and to perform positioning of the balloon again, manipulation is complicated.

Therefore, to prevent the balloon from slipping on and being displaced from the stenosis site or the like during inflation of the balloon, a treatment tool for an endoscope using a balloon in which a small-diameter portion is formed between a distal portion and a proximal portion when the balloon inflates has been proposed (for example, Patent Document 1).

In the treatment tools for an endoscope of Patent Documents 1 to 3, a radiopaque marker is provided on a guide member such as a tube inserted into the balloon in order to dispose the small-diameter portion in the stenosis site or the like. In these treatment tools for an endoscope, a position of the placed balloon is specified under observation of radioscopy while checking a position of the marker.

Patent Document 4 discloses a balloon catheter including an elongated shaft having an inner tube and an outer tube, and a balloon, provided at a distal end of the shaft and configured to be inserted into a stenotic portion. A first contrast marker is provided on a distal side of the inner tube and indicates a position of a maximum outer diameter portion of a distal-side expansion portion, while a second contrast marker is provided on a proximal side of the inner tube and indicates a position of a maximum outer-diameter portion of a proximal-side expansion portion. A further contrast marker is provided on the inner tube at an intermediate position between the first and second contrast markers, in the proximity of a center position of an expansion effective portion. The contrast markers may be realized in a radiopaque material.

Patent Document 5 discloses a dilation balloon catheter comprising a balloon distally mounted on a catheter, the catheter including a wire guide which extends through the balloon in the longitudinal direction. A plurality of markers are provided on the wire guide for enabling the operator orienting the balloon to a desired location in the human body. Each of the markers is provided on a respective section of the balloon. Specifically, a first marker (e.g., comprising a triple band) identifies a center portion or waist of a distal balloon section, a second marker (e.g., comprising a double band) identifies an intermediate balloon section, and a third marker (e.g., comprising a single band) identifies a center portion or waist of a proximal balloon section. The markers can be made of a radiopaque material.

Patent Document 6 discloses a dilation catheter device for dilating an opening in a paranasal sinus and/or other passageways within the ear, nose or throat, comprising an elongate catheter shaft and an expandable dilator such as a balloon, mounted on a distal section of the shaft. Visible markers may be formed on a proximal end of the balloon and have a dark color (e.g., black or blue), so as to contrast with the pink color of the nasal mucosa and therefore be easily visible within the nose. Direct visualization markers may be formed on the catheter shaft and have a light color so as to contrast with the dark color of the catheter shaft, thereby allowing for easy viewing of the markers even in low light and operation conditions.

### [Prior Art Documents]

[Patent Document 1]
   Published Japanese Translation No. 2003-507906 of the PCT International Publication
[Patent Document 2]
   Published Japanese Translation No. 2008-528236 of the PCT International Publication
[Patent Document 3]
   Japanese Unexamined Patent Application, First Publication No. 2001-190678
[Patent Document 4]
   International Patent Application Publication No. 2015/146259 A1
[Patent Document 5]
   U.S. Patent No. 6 488 653 B1
[Patent Document 6]
   U.S. Patent Application Publication No. 2009/312745 A1

### [Disclosure of the Invention]

### [Problem to be solved by the Invention]

In Patent Documents 1 to 3, the position of the balloon is checked while checking the radiopaque marker under radioscopy. For this reason, the entire balloon can be checked from a side of the balloon (a direction orthogonal to a longitudinal axis of the tube), and the positions of the stenosis site or the like, the marker, and the balloon are easily found. However, a procedure under radioscopy is required.

In Patent Document 1, an example in which markings that can be seen with the endoscope are provided at middle portions of sections of the balloon is disclosed. However, an imager of the endoscope is provided at a distal portion of an endoscope insertion part, and picks up an image of the balloon, which is projected from a distal end of the endoscope insertion part, from the vicinity of a proximal end of the balloon. For this reason, in the case where the middle portions of the sections of the balloon are aligned with the stenosis site or the like prior to expansion of the balloon, the markings are concealed by the stenosis site or the like, and positions thereof cannot be specified. As a result, a user cannot see whether the small-diameter portion of the balloon is excessively pushed closer to a distal side than the stenosis site or the like. Since the image of the endoscope is a two-dimensional image, and the balloon is advanced/retracted in a depth direction of the image of the endoscope, it is difficult to see how much the balloon is advanced/retracted.

In view of the above circumstances, the present invention is directed to providing a treatment tool for an endoscope capable of easily disposing a balloon at an adequate position without using radioscopy or the like while checking an image acquired by an imager of the endoscope.

### [Means for Solving the Problems]

A treatment tool for an endoscope according to a first aspect of the present invention includes: a sheath; a balloon provided at a distal end of the sheath, formed of a film of a transparent material, and configured to be expandable from a folded initial shape to an unfolded inflated shape; an inner shaft connected to the distal end of the sheath and configured to protrude from a distal end of the balloon through an inside of the balloon; a middle marker configured to indicate a middle position of the balloon in a direction of a length of the balloon; and at least one or more positioning markers provided on the inner shaft independently of the middle marker and configured to be visible through the film of the balloon. The balloon has a proximal end-side region which is positioned at a proximal end part of the balloon and expansion of which is initiated when an internal pressure of the balloon is a first internal pressure value, a distal end-side region which is positioned at a distal end part of the balloon and inflation of which is initiated when the internal pressure of the balloon is the first internal pressure value, and a middle part that is provided between the proximal end-side region and the distal end-side region. When the internal pressure of the balloon is a first internal pressure value, the proximal end-side region and the distal end-side region undergo faster progress of unfolding than the middle part, and thereby the proximal end-side region and the distal end-side region have a larger diameter than the middle part, and when the internal pressure of the balloon is a second internal pressure value greater than the first internal pressure value, the proximal end-side region, the distal end-side region, and the middle part are deformed to the inflated shape, and a diameter of the middle part is configured to be substantially the same as those of the proximal end-side region and the distal end-side region. The positioning marker positioned at a most distal end is provided at a position corresponding to a predetermined range close to a proximal end from a boundary between the middle part and the proximal end-side region at a side closer to the proximal end than the middle part in a direction of a longitudinal axis of the inner shaft.

The positioning marker may be visible by an imager provided at a distal end of an endoscope insertion part through which the sheath is inserted.

The positioning marker may be visible by the imager in a state in which the balloon is folded.

When the balloon is folded in the initial shape, the balloon may have a plurality of wings being formed by folding the balloon, and the plurality of wings being wound around an axis of the balloon.

### [Effects of Invention]

According to the treatment tool for an endoscope of the present invention, a balloon is capable of being easily disposed at a suitable position while checking an image acquired by an imager of the endoscope.

### [Brief Description of Drawings]

Fig. 1 is a sectional view illustrating a treatment tool for an endoscope according to an embodiment of the present invention.
Fig. 2 is a view illustrating a state of a balloon in an initial shape in the treatment tool for an endoscope according to the embodiment of the present invention.
Fig. 3 is a view schematically illustrating a cross section of the initial shape of the balloon in a direction orthogonal to a longitudinal axis.
Fig. 4 is a view illustrating a use state of the treatment tool for an endoscope according to the embodiment of the present invention.
Fig. 5 is a view illustrating an operation of the treatment tool for an endoscope according to the embodiment of the present invention when the treatment tool for an endoscope is used.
Fig. 6 is a view illustrating an image example of an imager of an endoscope when the treatment tool for an endoscope according to the embodiment of the present invention is used.
Fig. 7 is a view illustrating an operation of the treatment tool for an endoscope according to the embodiment of the present invention when the treatment tool for an endoscope is used.
Fig. 8 is a view illustrating an operation of the treatment tool for an endoscope according to the embodiment of the present invention when the treatment tool for an endoscope is used.
Fig. 9 is a view illustrating an operation of the treatment tool for an endoscope according to the embodiment of the present invention when the treatment tool for an endoscope is used.

### [Description of Embodiments]

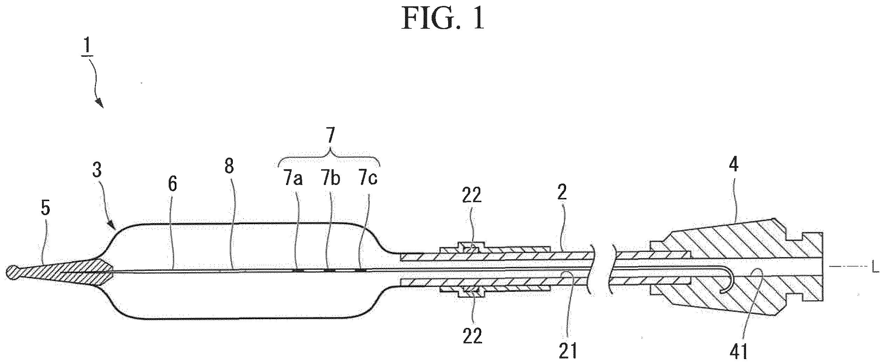

An embodiment of the present invention will be described with reference to Figs. 1 to 9. Fig. 1 is a sectional view illustrating a treatment tool 1 for an endoscope of the present embodiment. The treatment tool 1 for an endoscope includes a sheath 2, a balloon 3, a connector 4, a distal end tip 5, a stylet (an inner shaft) 6, a positioning marker 7, and a middle marker 8.

The sheath 2 is a long, flexible member that has a lumen 21 and extends in a direction of a longitudinal axis L. A proximal portion of the balloon 3 is airtightly connected to a distal portion of the sheath 2. The connector 4 is provided at a proximal portion of the sheath 2. A communication passage 41 that communicates from a distal end to a proximal end along the longitudinal axis L is formed in the connector 4. The lumen 21 of the sheath 2 communicates with an inside of the balloon 3 and the communication passage 41 of the connector 4. Therefore, the balloon 3 is capable of being enlarged in a diameter by supplying a fluid to the balloon 3 via the communication passage 41 and the lumen 21 by a syringe or the like connected to the connector 4.

A marker 22 being capable of being visualized under radioscopy is provided at the distal portion of the sheath 2 in order to facilitate the check of a position of a terminal portion of the proximal portion of the balloon.

The balloon 3 is a bag-shaped member formed of, for example, a transparent film made of a polyamide resin (for example, PEBAX (registered trademark) manufactured by ARKEMA Inc.).

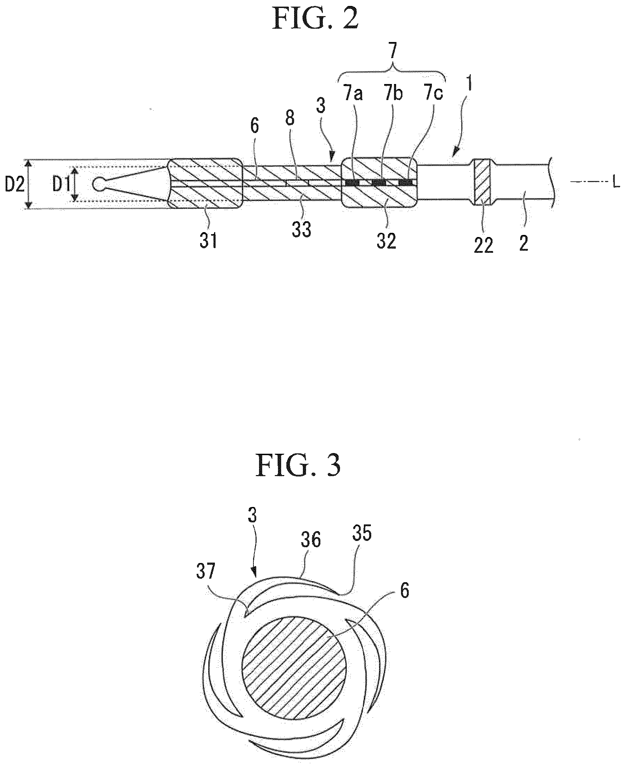

An initial shape that is a shape of the balloon 3 prior to inflation is illustrated in Fig. 2. A cross section of the balloon 3 having the initial shape in the direction orthogonal to the longitudinal axis L is schematically illustrated in Fig. 3. The balloon 3 is folded by molding a material in an approximately cylindrical shape and then forming folds such that a plurality of wings 36 extending in the direction of the longitudinal axis L are formed. In this case, a plurality of valleys 37 (see Fig. 3) formed to protrude inward in a radial direction at root sides of the wings 36 are formed. A shape of the balloon in which a diameter is reduced by this folding is referred to as an initial shape. Details of the initial shape will be described below.

When a fluid is injected to the balloon 3, the wings are spread, and thereby the balloon 3 opens while being unfolded, and inflates to be in an approximately columnar shape. In this way, a shape that is nearly inflated only by unfolding is referred to as an inflated shape. The balloon 3 is reduced in internal volume so that a size thereof in a radial direction is capable of being contracted, and ideally returns to a shape close to the initial state by removing the fluid injected into the balloon 3 of the inflated shape. When an internal pressure in the balloon 3 of the inflated shape reaches or exceeds a predetermined value, the balloon 3 is expanded and enlarged in diameter while the constituted film is stretched. That is, the balloon 3 is a so-called semi-compliant type balloon.

As illustrated in Fig. 2, the balloon 3 has a distal end-side region 31 adjacent to the distal end tip 5, a proximal end-side region 32 adjacent to the sheath 2, and a middle part 33 between the distal end-side region 31 and the proximal end-side region 32. As illustrated in Fig. 3, in the initial shape, the balloon 3 is folded such that the balloon 3 is bent to form the plurality of wings 36 that protrude outward in a radial direction and the plurality of wings 36 is wound around an axis of the balloon 3. Outer diameters D2 of the distal end-side region 31 and the proximal end-side region 32 that are located at opposite ends of the balloon 3 in a longitudinal direction (that is the same as an axial direction of the balloon 3) are larger than an outer diameter D1 of the middle part 33.

In the balloon 3 of the initial shape, an amount of residual strain in the distal end-side region 31 and the proximal end-side region 32 is different from that at the middle part 33 due to a difference of an amount of deformation of a film generated by a folding process. In this specification, "amount of residual strain" refers to the total sum of the amounts of residual strain within a range of a predetermined unit length in the axial direction of the balloon. In the case where a certain region has a length larger than or equal to the unit length in the axial direction, the amount of residual strain per unit length calculated by averaging the amounts of residual strain of the parts is set as the amount of residual strain of the region.

In the balloon 3 of the initial shape, residual strain is exclusively generated at portions of folding lines 35 that are tops of the wings 36, and at the valleys 37 that are bent in the middle between pairs of neighboring wings 36 to be convexedly bent toward the stylet 6. In the balloon 3 of the present embodiment, an amount of deformation of the middle part 33 is made larger than those of the distal end-side region 31 and the proximal end-side region 32. As an amount of deformation generated at the film of which the balloon 3 is formed by the folding process increases, the residual strain generated at the folding lines 35 and the valleys 37 becomes larger. Thus, in the balloon 3 of the present embodiment, the amount of residual strain at the middle part 33 is configured to become larger than that in the distal end-side region 31 and the proximal end-side region 32.

As illustrated in Fig. 2, the distal end tip 5 is provided at a distal end of the treatment tool 1 for an endoscope. The distal end tip 5 is a member that extends in the direction of the longitudinal axis L and has an approximately conical shape, and a distal portion of the distal end tip 5 is formed in a spherical shape to prevent damage to tissue when the distal end tip 5 is inserted into a body cavity.

A distal portion of the balloon 3 is closely fixed to a proximal portion of the distal end tip 5.

The stylet 6 is a shaft member. The stylet 6 is inserted into the balloon 3, and runs from a distal end to a proximal end of the balloon 3 along the longitudinal axis L. A distal portion of the stylet 6 is connected to a proximal end of the distal end tip 5. The stylet 6 extends through the inside of the balloon 3, the lumen 21 of the sheath 2, and the communication passage 41 of the connector 4, and is fixed to an inner wall of the communication passage 41 of the connector 4. The stylet 6 is formed of, for example, stainless steel, a nickel-titanium alloy, or the like.

The positioning marker 7 (hereinafter, sometimes simply referred to as "marker 7") is visible through the film of the balloon 3, and a plurality of markers 7 are provided on the stylet 6. In the present embodiment, the positioning marker 7 is made up of a first marker 7a, a second marker 7b, and a third marker 7c that are provided at intervals in order from the vicinity of the distal end.

The number of positioning markers 7 is not limited to the above, and at least one or more may be provided. For example, the number of positioning markers 7 may be increased/decreased depending on an effective length of the balloon or a position of a boundary between the middle part 33 and the proximal end-side region 32. For example, the number of positioning markers 7 may be only one.

At a side more proximally than the middle part in the direction of the longitudinal axis L of the stylet 6, the first marker 7a located at a most distal end among the three positioning markers 7 is provided on the stylet 6 at a position corresponding to a predetermined range that is a same longitudinal position as the boundary between the middle part 33 and the proximal end-side region 32 to proximal side. For example, in a case in which an effective length of the balloon is 80 millimeters (mm), the predetermined range from a longitudinal position of the boundary between the middle part 33 and the proximal end-side region 32 to the proximal side is a range which is a same longitudinal potion as the boundary between the middle part 33 and the proximal end-side region 32 and is a range of about 2 to 3 mm from the boundary. Therefore, a distal end of the first marker 7a may be located at a same longitudinal position as the boundary between the middle part 33 and the proximal end-side region 32 (at a proximal end of the middle part 33), or may be located at a same longitudinal position as a proximal side of the balloon where is more proximal than the boundary between the middle part 33 and the proximal end-side region 32. This range is not limited to the above, and the range may be changed depending on the effective length of the balloon or a position of the boundary between the middle part 33 and the proximal end-side region 32.

In the present embodiment, the second marker 7b and the third marker 7c are provided at a proximal side from the first marker 7a at a pitch of 10 mm. The plurality of markers 7 are disposed at a distal side from at a position at which the proximal end of the balloon 3 is joined with the stylet 6 in consideration of a depth of a field of view of the imager 153. The first marker 7a is positioned on the stylet at same longitudinal position as around the boundary between the middle part 33 and the proximal end-side region 32, and the three markers 7a, 7b and 7c are provided at a regular pitch.

The second marker 7b and the third marker 7c are provided so that an amount of advancing/retracting of the stylet 6 and a position of the first marker 7a is capable of being easily seen. The three markers 7a, 7b and 7c are intermittently provided, and thereby a user is capable of easily seeing an amount of advancing/retracting of the balloon 3.

The positioning marker 7 may be provided, for example, by painting an outer surface of the stylet 6 with paint or putting a tape on the stylet 6. In addition, the positioning marker may be provided by inserting a tubular member around the stylet 6, thermally contracting the tubular member, and externally fitting the tubular member. In the case where the tubular member is mounted, a proximal end face of the positioning marker formed by a thickness of the tubular member becomes a reflecting surface of illumination light 154 of the endoscope 150, and visibility is capable of being improved more than with the positioning marker of paint or tape.

When colors of the markers 7 are selected from colors that are complementary colors to a color of a surface of tissue T, this is preferred because a contrast with the tissue T that is a background within the field of view of the endoscope 150 is high, and visibility of the markers 7 is relatively improved. The colors of the markers 7 need only be visible via the imager 153 of the endoscope 150, and may be selected within a range overlapping the color of the surface of the tissue in addition to the colors different from the color of the surface of the tissue T.

The middle marker 8 is a marker that indicates a position of the middle part 33 and is provided at a position corresponding to the middle part 33 on the stylet 6 in the direction of the longitudinal axis L. Therefore, the middle marker 8 and the three positioning markers 7a, 7b and 7c are provided on the stylet 6 from the vicinity of the distal end in turn. Like the positioning marker 7, the middle marker 8 is visible under observation of the endoscope. The middle marker 8 may also be the same color as the positioning marker 7. However, the middle marker 8 may have a different color or shape from the positioning marker 7 in order to be easily distinguished from the positioning marker 7 during manipulation. In addition, the middle marker may be provided on a surface of the middle part 33 of the balloon 3.

The middle marker 8 is visible until the middle part 33 enters a stenosis site or the like, and is not visible after the middle part 33 enters the stenosis site or the like. However, an operator is capable of easily seeing the relative positional relationship between the middle part of the balloon and a middle part of the stenosis with the help of a position, a moving direction, and an amount of movement of the positioning marker 7 within the field of view of the endoscope according to an interval distance between the middle marker 8 and the positioning marker 7. Even in this case, the positioning marker 7 at the most distal end is the first marker 7a.

An operation of the treatment tool 1 for an endoscope configured in this way when the treatment tool 1 is used will be described.

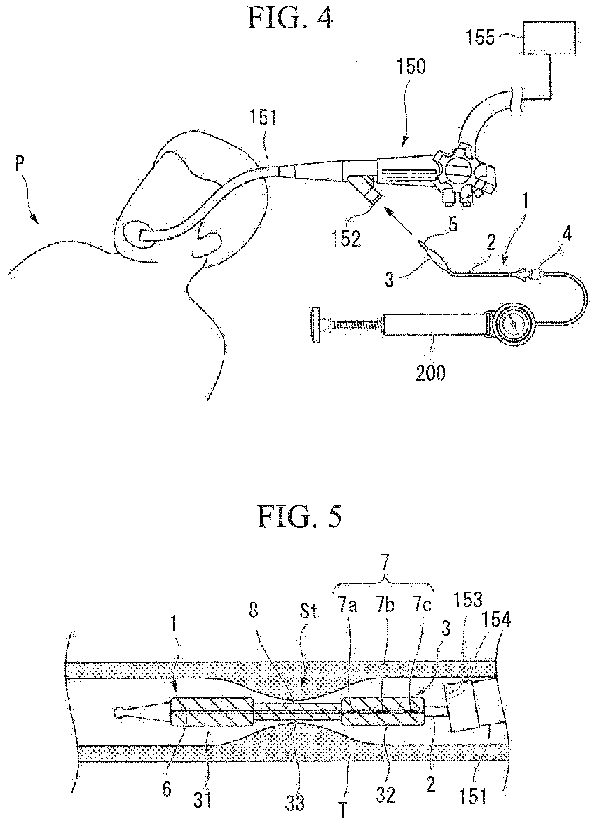

As illustrated in Fig. 4, the treatment tool 1 for an endoscope is introduced into the body of a patient via a channel provided in an insertion part of the endoscope. As illustrated in Fig. 4, a user connects an inflator 200 to the connector 4, and inserts the treatment tool 1 for an endoscope into an insertion part 151 from a forceps opening 152 of the endoscope 150. Afterward, the endoscope 150 is inserted into the body of the patient P. The distal end of the endoscope 150 is advanced to the vicinity of a site on which an expansion procedure is performed, for example, a predetermined site of the esophagus. The connection between the treatment tool 1 for an endoscope and the inflator 200 or the insertion of the treatment tool 1 for an endoscope into the endoscope 150 may be performed after the endoscope 150 is inserted into the body of the patient P.

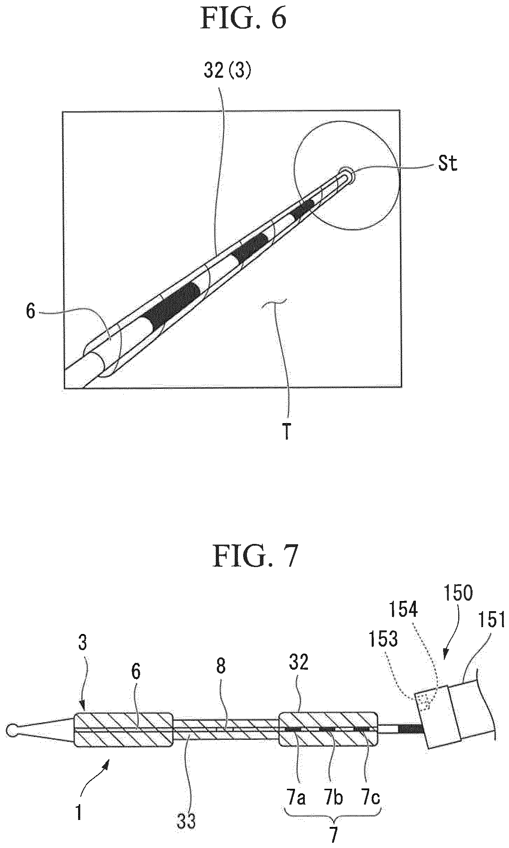

The user causes the treatment tool 1 for an endoscope to protrude from the endoscope 150 while observing the target site on which the expansion procedure is performed by the endoscope 150 while using a display unit 155 of the endoscope 150, and inserts the distal end tip 5 into the target site. As illustrated in Fig. 7, a versatile imager 153 disposed close to the proximal portion of the balloon 3 images an inlet of the stenosis site or the like located far from the field of view. In this case, a distal end of the endoscope insertion part is essentially located in a direction that intersects the longitudinal axis L, and the imager 153 faces the stylet 6.

The user further advances the treatment tool 1 for an endoscope until the middle marker 8 enters the target site and is not seen by the display unit 155 of the endoscope 150. In this case, the user checks the three markers 7a, 7b and 7c through the display unit 155, and can see the amount of advancing/retracting of the balloon 3.

Fig. 6 is a view illustrating an image example of the imager 153 of the endoscope 150 when the treatment tool 1 for an endoscope is used. As illustrated in Figs. 5 and 6, a farthest visible area which viewed by an image through the imager 153 is an opening end positioned at a proximal side of the target site St of the stenosis site or the like, and an area more distal than the stenosis site or the like is not visible. For this reason, after the middle marker 8 enters the target site St, the middle marker 8 is not visible. However, since the first marker 7a is provided at more proximally in a longitudinal position than the boundary between the middle part 33 and the proximal end-side region 32, the user is capable of recognizing the position of the middle part 33 relative to the target site St by means of a position of the first marker 7a.

As illustrated in Fig. 7, the imager 153 picks up the image of the inlet of the stenosis site or the like located far in the field of view from the vicinity of the proximal portion of the balloon 3. For this reason, as illustrated in Fig. 6, it is difficult to see a distance around the inlet of the stenosis site or the like in a two-dimensional image of the imager 153. However, because the three markers 7a, 7b and 7c are intermittently provided, the amount and the distance of advancing/retracting of the balloon 3 is easily seen.

For example, in the case where the distance of the field of view of the endoscope 150 is about 30 mm, when the first marker 7a is separated from the imager 153 of the endoscope 150, which is disposed close to the proximal end of the balloon 3, to the distal side by 55 mm or more at the imager 153 of the endoscope 150, it is difficult to see the position of the first marker 7a through the image obtained by the imager 153. In this way, even when the position of the first marker 7a in a depth direction is difficult to see, the position of the middle part 33 is easily adjusted in the direction of the longitudinal axis L by the positions of the second and third markers 7b and 7c that are intermittently disposed at more proximal side than the first marker 7a.

Due to the above manipulation, the treatment tool 1 for an endoscope is held such that the distal end-side region 31 of the balloon 3 is located at more distal than the target site and the proximal end-side region 32 of the balloon 3 is located at more proximal than the target site.

Next, the user operates the inflator 200 to supply a fluid such as water or air to the balloon 3. The balloon 3 is inflated while raising an internal pressure due to the supplied fluid, but the middle part 33 has a larger amount of residual strain than the distal end-side region 31 and the proximal end-side region 32. Thus, a great force is required such that the wings 36 are inflated by straightening the folding lines 35 and the valleys 37 in a linear shape.

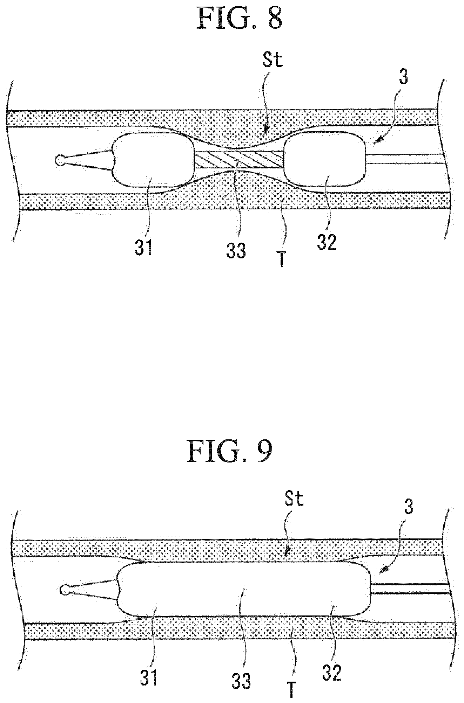

In the state in which the internal pressure of the balloon 3 reaches a predetermined first internal pressure value P1 due to the supply of the fluid, the distal end-side region 31 and the proximal end-side region 32 undergo faster progress of unfolding than the middle part 33. Even in a semi-inflated state in which the unfolding of the balloon 3 is in progress, the user can adjust a position of the middle of the balloon 3 while checking the positions of the markers 7. Since the progress of the unfolding is slow, the middle part 33 is smaller in diameter than the distal end-side region 31 and the proximal end-side region 32 (the distal end-side region 31 and the proximal end-side region 32 getting larger in diameter than the middle part 33), and the balloon 3 is deformed to a dumbbell shape as a whole as illustrated in Fig. 8. Therefore, even if the balloon 3 slips due to mucus or the like of a surface of a lumen organ, the distal end-side region 31 and the proximal end-side region 32 serve as anchors, and are inhibited from moving, and a situation, for example, in which the balloon 3 is removed from the target site St is adequately prevented.

When the internal pressure of the balloon 3 reaches a second internal pressure value P2 that is higher than the first internal pressure value P1, all of the distal end-side region 31, the proximal end-side region 32, and the middle part 33 are unfolded, and the balloon 3 is nearly returned to the approximately cylindrical shape from before the folding process was performed as illustrated in Fig. 9 (the inflated shape). In this case, the distal end-side region 31 and the proximal end-side region 32 have the same dimension or substantially the same dimension in a radial direction as the middle part 33. The middle part 33 is inflated to substantially the same diameter as the distal end-side region 31 and the proximal end-side region 32, and thereby the target site St is capable of being sufficiently dilated.

The film of which the balloon 3 is formed is hardly stretched at the second internal pressure value P2. However, since the balloon 3 is a semi-compliant type, if the internal pressure is increased above a third internal pressure value P3 higher than the second internal pressure value as needed, the entire balloon 3 is further inflated while stretching the material, and a great expansion force is also capable of being applied by the target site St.

In more detail, since the material of which the balloon 3 is formed is hardly stretched until the internal pressure of the balloon 3 reaches the second internal pressure value P2, the balloon 3 is inflated depending exclusively on the progress of the unfolding, and outer diameters of the distal end-side region 31, the proximal end-side region 32, and the middle part 33 increase.

Since the folding is nearly released after the internal pressure of the balloon 3 reaches the second internal pressure value P2, an increase in outer diameter hardly occurs even if the internal pressure rises. When the internal pressure of the balloon 3 further rises and is increased above the third internal pressure value P3, although the film material of which the balloon 3 is formed starts stretching but the expansion caused by the progress of the unfolding hardly occurs. Therefore, the outer diameters of the distal end-side region 31, the proximal end-side region 32, and the middle part 33 increase depending exclusively on the stretching of the film material. The balloon 3 is formed in an inflated shape, and thereby the stenosis site is spread.

As described above, according to the treatment tool 1 for an endoscope of the present embodiment, since the positioning marker 7 is provided on the stylet 6 at the position corresponding to the proximal end-side region 32 of the balloon 3, the position of the middle part 33 of the balloon 3 is capable of being easily seen even after the middle part 33 of the balloon 3 enters the stenosis site or the like. It is possible to prevent the balloon 3 from being inflated in the state in which the balloon 3 is excessively advanced to the distal side and the middle part 33 is disposed at more distal than the stenosis site or the like. Therefore, the middle part 33 is capable of being disposed at a suitable position of the stenosis site or the like by smooth manipulation. As a result, the balloon 3 is possible to hardly slip on the stenosis site or the like during the inflation of the balloon 3.

According to the treatment tool 1 for an endoscope of the present embodiment, since the position of the balloon 3 is capable of being seen by using the positioning marker 7 that is visible via the imager 153 of the endoscope 150, the treatment tool is excellent in terms of user convenience.

According to the treatment tool 1 for an endoscope of the present embodiment, the balloon is capable of being disposed at a more suitable position with respect to the target site, and an effect of preventing position displacement is capable of being positively exhibited.

The treatment tool 1 for an endoscope according to the present embodiment is particularly effective, for example, in the case where the length of the stenosis site is long.

Since the positioning marker 7 is visible through the film in the initial shape in which the balloon 3 is folded, there is no need to inflate the balloon 3 prior to performing the positioning, and positioning manipulation is capable of being smoothly performed.

According to the treatment tool 1 for an endoscope of the present embodiment, the amount of residual strain of the middle part 33 in the balloon 3 is set to be greater than those of the distal end-side region 31 and the proximal end-side region 32 that are disposed to sandwich the middle part 33. As a result, the balloon 3 is formed in the dumbbell shape in which the distal end-side region 31 and the proximal end-side region 32 are inflated to have the outer diameters larger than that of the middle part 33 at the first internal pressure value P1, and the balloon is capable of being appropriately prevented from being removed or displaced from the target site St in a process of treatment at the target site St.

The middle part 33 is capable of being inflated to substantially the same diameter as the distal end-side region 31 and the proximal end-side region 32 at the second internal pressure value P2, and the target site St is capable of being sufficiently dilated.

As a result, the prevention of position displacement against the target site and the sufficient expansion of the target site are compatible, and expansion treatment can be suitably performed on the target site of the stenosis site or the like.

In the present embodiment, no marker is provided at the position corresponding to the distal end-side region 31 in the direction of the longitudinal axis L of the stylet 6. For the purpose of disposing the middle part 33 at the stenosis site or the like, since the more distal end side than the distal end than the middle part 33 is not visible by the imager 153 after the middle part 33 enters the stenosis site or the like, no marker is required. No marker is provided in the distal end-side region 31 in order to prevent confusion with the positioning marker 7 by providing an unnecessary marker.

In the present embodiment, the first internal pressure value and the second internal pressure value may be set to desired values by appropriately setting the amounts of residual strain of the distal end-side region 31, the proximal end-side region 32, and the middle part 33. The second internal pressure value need only be set on the basis of a pressure that is desired to be applied to the target site, and the second internal pressure may be set to, for example, ca. 30,4 kPA (three atmospheres (atm)). The first internal pressure value is preferably set to be sufficiently lower than the second internal pressure value such that the effect of preventing position displacement is exerted early, and the first internal pressure may be set to, for example, ca. 50,66 kPa (0,5 atm).

In the above example, an example in which the amounts of residual strain of the distal end-side region 31 and the proximal end-side region 32 are made different from that of the proximal end-side region 32 by making the outer diameters of the distal end-side region 31 and the proximal end-side region 32 in the initial shape to be different from that of the middle part 33 has been described, but a method of making both of the amounts of residual strain different from each other is not limited thereto. For example, it is possible that the amount of residual strain of the middle part 33 is also relatively increased while making the outer diameters of the distal end-side region 31, the proximal end-side region 32, and the middle part 33 in the initial shape the same or substantially the same.

In the present embodiment, an example in which the three markers 7 are provided has been described, but two or more markers 7 may be intermittently provided. However, when there are too many markers, the pitch for the markers is essentially made fine. When the pitch for the markers is made excessively fine in the case where the marker is observed at a shallow angle with respect to the balloon 3, it is difficult to find out the advance/retract and the position of the marker. Thus, for example, a sufficient number to dispose the markers having a length of 2 mm to 4 mm in the direction of the longitudinal axis L at a pitch of 10 mm need only be provided according to the length of the proximal end-side region.

In the aforementioned embodiment, an example in which the balloon is the semi-compliant type has been described, but a so-called non-compliant type balloon in which the material of which the balloon is formed is not substantially stretched even if the internal pressure is greater than or equal to the second internal pressure value may be used.

### [Industrial Applicability]

According to the treatment tool for an endoscope, the balloon can be easily disposed at a suitable position while checking the image acquired by the imager of the endoscope.

### [Reference Signs List]

1 Treatment tool for endoscope
2 Sheath
3 Balloon
7, 7a, 7b, 7c Positioning marker
8 Middle marker
31 Distal end-side region
32 Proximal end-side region
33 Middle part

## Claims

1. A treatment tool (1) for an endoscope (150), comprising:
a sheath (2);
a balloon (3) provided at a distal end of the sheath (2), formed of a film of a transparent material, and configured to be expandable from a folded initial shape to an unfolded inflated shape;
an inner shaft (6) connected to the distal end of the sheath (2) and configured to protrude from a distal end of the balloon (3) through an inside of the balloon (3);
a middle marker (8) configured to indicate a middle position of the balloon (3) in a direction of a length of the balloon (3); and
at least one or more positioning markers (7, 7a, 7b, 7c) provided on the inner shaft (6) independently of the middle marker (8) and configured to be visible through the film of the balloon (3),
wherein the balloon (3) has:
a proximal end-side region (32) which is positioned at a proximal end part of the balloon (3) and inflation of which is initiated when an internal pressure of the balloon (3) is a first internal pressure value;
a distal end-side region (31) which is positioned at a distal end part of the balloon (3) and inflation of which is initiated when the internal pressure of the balloon (3) is the first internal pressure value; and
a middle part (33) that is provided between the proximal end-side region (32) and the distal end-side region (31),
wherein when the internal pressure of the balloon (3) is a first internal pressure value, the proximal end-side region (32) and the distal end-side region (31) undergo faster progress of unfolding than the middle part (33), and thereby the proximal end-side region (32) and the distal end-side region (31) have a larger diameter (D2) than the middle part (33), and
when the internal pressure of the balloon (3) is a second internal pressure value greater than the first internal pressure value, the proximal end-side region (32), the distal end-side region (31), and the middle part (33) are deformed to the inflated shape, and a diameter (D1) of the middle part (33) is configured to be substantially the same as those of the proximal end-side region (32) and the distal end-side region (31), and
wherein the positioning marker (7a) positioned at a most distal end is provided at a position corresponding to a predetermined range close to a proximal end from a boundary between the middle part (33) and the proximal end-side region (32) at a side closer to the proximal end than the middle part (33) in a direction of a longitudinal axis (L) of the inner shaft (6).

2. The treatment tool (1) for an endoscope (150) according to Claim 1, wherein the positioning marker (7, 7a, 7b, 7c) is visible by an imager (153) mounted at a distal end of an endoscope insertion part (151) through which the sheath (2) is inserted.

3. The treatment tool (1) for an endoscope (150) according to Claim 2, wherein the positioning marker (7, 7a, 7b, 7c) is visible by the imager (153) in a state in which the balloon (3) is folded.

4. The treatment tool (1) for an endoscope (150) according to Claim 1, wherein when the balloon (3) is folded in the initial shape, the balloon (3) has a plurality of wings (36) being formed by folding the balloon (3), and the plurality of wings (36) being wound around an axis of the balloon (3).

## Patentansprüche

1. Behandlungswerkzeug (1) für ein Endoskop (150), umfassend:
eine Hülle (2);
einen Ballon (3), der an einem distalen Ende der Hülle (2) vorgesehen ist, aus einer Folie aus einem transparenten Material besteht und so konfiguriert ist, dass er von einer gefalteten Ausgangsform in eine entfaltete, aufgeblasene Form expandierbar ist;
einen inneren Schaft (6), der mit dem distalen Ende der Hülle (2) verbunden und so konfiguriert ist, dass er von einem distalen Ende des Ballons (3) durch das Innere des Ballons (3) herausragt;
eine Mittelmarkierung (8), die so konfiguriert ist, dass sie eine mittlere Position des Ballons (3) in einer Richtung der Länge des Ballons (3) anzeigt; und
mindestens eine oder mehrere Positionierungsmarkierungen (7, 7a, 7b, 7c), die unabhängig von der Mittelmarkierung (8) auf dem inneren Schaft (6) vorgesehen und so konfiguriert sind, dass sie durch die Folie des Ballons (3) sichtbar sind,
wobei der Ballon (3) aufweist:
einen proximalen endseitigen Bereich (32), der an einem proximalen Endteil des Ballons (3) angeordnet ist und dessen Aufblasen eingeleitet wird, wenn ein Innendruck des Ballons (3) einen ersten Innendruckwert beträgt;
einen distalen endseitigen Bereich (31), der an einem distalen Endteil des Ballons (3) angeordnet ist und dessen Aufblasen eingeleitet wird, wenn der Innendruck des Ballons (3) den ersten Innendruckwert beträgt; und
ein Mittelteil (33), das zwischen dem proximalen endseitigen Bereich (32) und dem distalen endseitigen Bereich (31) vorgesehen ist,
wobei, wenn der Innendruck des Ballons (3) ein erster Innendruckwert ist, der proximale endseitige Bereich (32) und der distale endseitige Bereich (31) einen schnelleren Fortschritt der Entfaltung erfahren als der mittlere Teil (33), und dadurch der proximale endseitige Bereich (32) und der distale endseitige Bereich (31) einen größeren Durchmesser (D2) haben als der Mittelteil (33), und
wenn der Innendruck des Ballons (3) ein zweiter Innendruckwert ist, der größer als der erste Innendruckwert ist, der proximale endseitige Bereich (32), der distale endseitige Bereich (31) und der Mittelteil (33) in die aufgeblasene Form verformt werden, und ein Durchmesser (D1) des Mittelteils (33) so konfiguriert ist, dass er im Wesentlichen derselbe ist wie der des proximalen endseitigen Bereichs (32) und des distalen endseitigen Bereichs (31), und
wobei die Positionierungsmarkierung (7a), die an einem distalsten Ende positioniert ist, an einer Position vorgesehen ist, die einem vorbestimmten Bereich nahe einem proximalen Ende von einer Grenze zwischen dem Mittelteil (33) und dem proximalen endseitigen Bereich (32) auf einer Seite näher am proximalen Ende als der Mittelteil (33) in einer Richtung einer Längsachse (L) des inneren Schafts (6) entspricht.

2. Behandlungswerkzeug (1) für ein Endoskop (150) nach Anspruch 1, wobei die Positionierungsmarkierung (7, 7a, 7b, 7c) durch einen Bildgeber (153) sichtbar ist, der an einem distalen Ende eines Endoskopeinführungsteils (151) angebracht ist, durch das die Hülle (2) eingeführt wird.

3. Behandlungswerkzeug (1) für ein Endoskop (150) nach Anspruch 2, wobei die Positionierungsmarkierung (7, 7a, 7b, 7c) in einem Zustand, in dem der Ballon (3) gefaltet ist, durch den Bildgeber (153) sichtbar ist.

4. Behandlungswerkzeug (1) für ein Endoskop (150) nach Anspruch 1, wobei, wenn der Ballon (3) in die Ausgangsform gefaltet ist, der Ballon (3) eine Vielzahl von Flügeln (36) aufweist, die durch Falten des Ballons (3) gebildet werden, und die Vielzahl von Flügeln (36) um eine Achse des Ballons (3) gewickelt sind.

## Revendications

1. Outil de traitement (1) pour un endoscope (150), comprenant :
une gaine (2) ;
un ballonnet (3) disposé à une extrémité distale de la gaine (2), constitué d'un film en matériau transparent, et conçu pour être expansible depuis une forme initiale pliée à une forme dépliée gonflée ;
un arbre interne (6) relié à l'extrémité distale de la gaine (2) et conçu pour faire saillie de l'extrémité distale du ballonnet (3) à travers l'intérieur du ballon (3) ;
un marqueur médian (8) conçu pour indiquer une position médiane du ballonnet (3) dans une direction d'une longueur du ballonnet (3) ; et
au moins un ou plusieurs marqueurs (7, 7a, 7b, 7c) disposés sur l'arbre interne (6) indépendamment du marqueur médian (8) et conçus pour être visibles à travers le film du ballonnet (3),
dans lequel le ballonnet (3) a :
une région côté extrémité proximale (32) qui est positionnée à une partie d'extrémité proximale du ballonnet (3) et qui commence à gonfler lorsqu'une pression interne du ballonnet (3) est une première valeur de pression interne ;
une région côté extrémité distale (31) qui est positionnée à une partie d'extrémité distale du ballonnet (3) et qui commence à gonfler lorsqu'une pression interne du ballonnet (3) est le première valeur de pression interne ; et
une partie médiane (33) qui est entre la région côté extrémité proximale (32) et la région côté extrémité distale (31),
dans lequel, lorsque la pression interne du ballonnet (3) est une première pression interne, la région côté extrémité proximale (32) et la région côté extrémité distale (31) sont soumises à une progression plus rapide de déploiement que la partie médiane (33), et ainsi la région côté extrémité proximale (32) et la région côté extrémité distale (31) ont un diamètre supérieur (D2) à la partie médiale (33) ; et
lorsque la pression interne du ballonnet (3) est une seconde valeur de pression interne supérieure à la première valeur de pression interne, la région côté extrémité proximale (32), la région côté extrémité distale (31) et la partie médiane (33) sont déformée en une forme gonflée, et un diamètre (D1) de la partie médiane (33) est conçu pour être sensiblement identique à ceux de la région côté extrémité proximale (32) et de la région côté extrémité distale (31), et
dans lequel le marqueur de positionnement (7a) positionné à l'extrémité la plus distale est disposé à une position correspondant à une portée prédéfinie proche d'une extrémité proximale d'une limite entre la partie médiane (33) et la région côté extrémité proximale (32) disposée à un côté plus proche de l'extrémité proximale que la partie médiane (33) dans une direction d'un axe longitudinal (L) de l'arbre interne (6).

2. Outil de traitement (1) pour un endoscope (150) selon la revendication 1, dans lequel le marqueur de positionnement (7, 7a, 7b, 7c) est visible par un imageur (153) monté à une extrémité distale d'une partie d'insertion d'endoscope (151) à travers lequel est insérée la gaine (2).

3. Outil de traitement (1) pour un endoscope (150) selon la revendication 2, dans lequel le marqueur de positionnement (7, 7a, 7b, 7c) est visible par l'imageur (153) dans un état dans lequel le ballonnet (3) est gonflé.

4. Outil de traitement (1) pour un endoscope (150) selon la revendication 1, dans lequel lorsque le ballonnet (3) est gonflée dans la forme initiale, le ballonnet (3) a une pluralité d'ailes (36) formées par pliage du ballonnet (3), et la pluralité d'ailes (36) étant enroulées autour d'un axe du ballonnet (3).
